Europäisches Patentamt

European Patent Office

Office européen des brevets

(11) Publication number: **0 332 018**
**A2**

(12) **EUROPEAN PATENT APPLICATION**

(21) Application number: **89103455.5**

(22) Date of filing: **28.02.89**

(51) Int. Cl.⁴: **G01N 21/31 , G01N 21/35 , G01N 33/34**

(30) Priority: **10.03.88 US 166278**

(43) Date·of publication of application:
**13.09.89 Bulletin 89/37**

(84) Designated Contracting States:
**DE FR GB IT SE**

(71) Applicant: **PROCESS AUTOMATION BUSINESS, INC.**
**650 Ackerman Road**
**Columbus Ohio 43202(US)**

(72) Inventor: **Sturm, Steven Perry**
**2104 Sumac Loop N.**
**Columbus Ohio 43229(US)**

(74) Representative: **Gross, Gernot K.**
**Rechtsanwalt**
**Kleiberweg 5**
**D-6200 Wiesbaden(DE)**

(54) Clay sensor.

(57) A sensor (2) for use in measuring the clay content of a sheet (8) of paper during its continuous manufacture. The sensor produces electrical indications of the intensity of radiation transmitted through or reflected from the sheet (8) for each of a plurality of selected narrow bands of infrared radiation. The selected bands include a measurement band having a center wavelength selected from a peak absorption range (46,48,50) for kaolin and a reference band having a center wavelength selected from outside a peak absorption range for kaolin. The radiation bands are selected to minimize error resulting from differences in sensitivity to absorption by water.

*FIG. 1*

## CLAY SENSOR

### BACKGROUND OF THE INVENTION

This invention relates generally to process automation within the paper industry and, more particularly, to sensors used in process automation systems. Specifically, the present invention pertains to infrared-based sensors for use in determining the amount of clay contained in a moving sheet of paper product.

It is a common practice in the paper industry to use sensors in measuring particular quality attributes of a sheet of paper product during its continuous manufacture. The measurements are derived from data which are provided by the sensors. The magnitudes of the data depend on various physical properties of the sheet such as transmittance, reflectance, and emittance. Known relationships between the physical property and the quality attribute of interest are employed in the design of front-end electronics and software to derive measurements of the quality attribute from the data provided by the sensors. Examples of such quality attributes are moisture content, basis weight, thickness, ash content, gloss, color, opacity, formation, and brightness.

One class of sensors (infrared-based sensors) employs the phenomenon of molecular resonance absorption to derive indications of the amount of a particular component which is contained in the paper. This class is most commonly used for deriving measurements of moisture content, although other applications such as the measurement of polymer content, fiber content are known. In a typical application, an indication of the content of a component is provided by determining the transmittance through or reflectance from the paper for two narrow bands of infrared radiation. One of these is typically designated a "measurement" or "absorption" band and is generally sensitive to absorption by the component of interest. The other is typically designated a "reference" band and is less sensitive to absorption by the same component. The ratio of the transmittances or reflectances of these two bands is related to the amount (typically, the weight per unit area) of the component which is contained in the paper. See U.S. Pat. Nos. 3,551,678 Mitchell, and 3,405,268 Brunton for examples of this approach. In the manufacture of some paper products, one or more filler materials are added to enable the manufacturer to meet standards for certain quality attributes at a lower cost than would result if the standards were met by increasing the amount of more expensive materials contained in the paper. One of these filler materials is clay. The type of clay ordinarily used in paper manufacturing is a hydrated aluminum silicate known as kaolin.

There is a limit to the amount of clay that can be added without sacrificing the ability to meet standards for paper strength. Accordingly, the ability to accurately measure the amount of clay is important in balancing manufacturing cost and quality.

Current approaches to measuring filler materials use ash sensors which derive indications of ash content (i.e. electrical signals from the sensor which are related to ash content, ash being used as a general term to describe a variety of combinations of clay, chalk, and titanium dioxide) via x-ray flourescence. These indications are sufficiently affected by water and cellulose to require the use of indications from one or more other sensors such as a moisture sensor and/or a beta gauge before an accurate measurement can be produced.

An objective of the present invention is to provide an on-line sensor which can be used to provide accurate measurements of the amount of clay contained in a moving sheet of paper.

Another objective of the invention is to provide such a sensor that is substantially insensitive to the amount of water contained in the moving sheet.

A further objective of the invention is to provide such a sensor that can be used to correct measurement error resulting from absorption and scattering effects produced by fiber and filler contained in the sheet.

### SUMMARY OF THE INVENTION

This invention provides a sensor for on-line use in measuring the amount of clay contained in a moving sheet of paper product.

The sensor provides electrical indications of the intensity of radiation transmitted through the sheet for each of a plurality of selected narrow bands of infrared radiation. The plurality of selected bands includes a first measurement band having a center wavelength selected from a peak absorption range for kaolin (hereinafter, the "kaolin measurement band") and first reference band having a center wavelength selected from outside a peak absorption range for kaolin (hereinafter the "kaolin reference band"). The plurality of selected bands may further include a second measurement band having a center wavelength that is sensitive to absorption by cellulose fiber (hereinafter the "fiber measurement band") and a second reference band having a center wavelength that is less sensitive to absorption by fiber.

The center wavelength of the kaolin reference band may be selected to have substantially the same sensitivity to absorption by water as the center wavelength of the kaolin measurement band so that error in the indications of clay content resulting from differential absorption by water is minimized. In addition, the center wavelength of the fiber reference band may be selected to have substantially the same sensitivity to absorption by water as the center wavelength of the fiber measurement band so that this error is substantially eliminated.

## BRIEF DESCRIPTION OF THE DRAWINGS

Fig. 1 is a generally schematic illustration of the preferred embodiment of the invention.

Fig. 2 is a graph illustrating absorption curves for water, cellulose, and kaolin over a spectral range of 1.0 to 3.6 microns. The ordinate is scaled for kaolin, but not for water or cellulose. However, the general shapes of the curves for water and cellulose are substantially accurate.

## DESCRIPTION OF THE PREFERRED EMBODIMENT

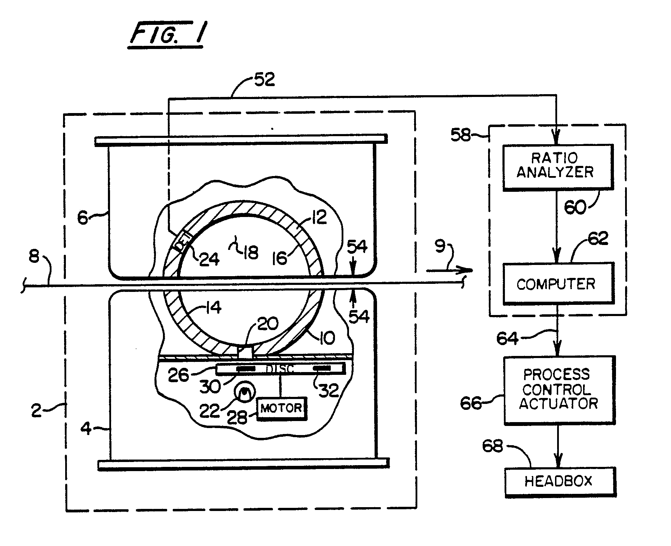

Referring to Fig. 1 the dashed line 2 represents a clay sensor which comprises a source housing 4 and a detector housing 6. In operation, the source and detector housings 4,6 are disposed on opposite sides of a moving sheet 8 of paper product which is being manufactured and which is predominantly composed of cellulose fiber, clay, and water. The sheet 8 may be viewed as moving in the direction indicated by the numeral 9.

The sensor 2 is similar in construction to that described in U.S. Pat. No. 4,052,615 Cho and in operation is mounted on a conventional sheet traversing structure (not shown) so that indications of clay content can be produced at several points across the width of the sheet 8. Rigidly secured to the source and detector housings 4,6 are hemispherical bodies 10,12. The hemispherical bodies 10,12 have oppositely facing, mirrorlike inner surfaces 14,16 which form a generally spherical cavity 18. A light pipe 20 is tightly fitted in a hole centered at the bottom of the hemispherical body 10 contained in the source housing 4. The center of the light pipe 20 defines a projection axis (not shown) extending from a radiation source 22 to the top of the other hemispherical body 12. A detector 24 is tightly fitted in a hole formed in the hemispherical body 12 and is positioned at about 60 degrees from the projection axis. The active element of the detector 24 is a lead sulfide photoresistor, although any photosensitive element which produces an electrical response to infrared radiation can be used in accordance with this invention.

The radiation source 22 may be any source which emits infrared over a spectral band which includes all narrow bands selected for practicing this invention, as further described below. A 375-watt tungsten halogen lamp (ANSI Code DWZ) operated at about 100 watts is preferred. A conventional filter wheel 26 is positioned so that optical filters installed therein are alternately passed between the source 22 and the light pipe 20. The filter wheel 26 is driven by a synchronous motor 28 contained in the source housing 4. Installed in the filter wheel 26 are four separate optical bandpass filters positioned at equally-spaced intervals around the wheel. The filters are selected to pass particular narrow bands of infrared radiation. The word "narrow" as used with the word "bands" herein is intended to convey that a bandpass filter selected to pass a narrow band of radiation has a half-power bandwidth of less than 0.108 microns.

A first filter 30 passes a first narrow band of measurement radiation which is sensitive to absorption by kaolin. Preferably, the center wavelength of kaolin measurement band is at about 2.18 microns. However, acceptable results can be obtained from any kaolin measurement band having a center wavelength selected either from the range extending from 2.15 to 2.22 microns or from the range extending from 1.38 to 1.42 microns.

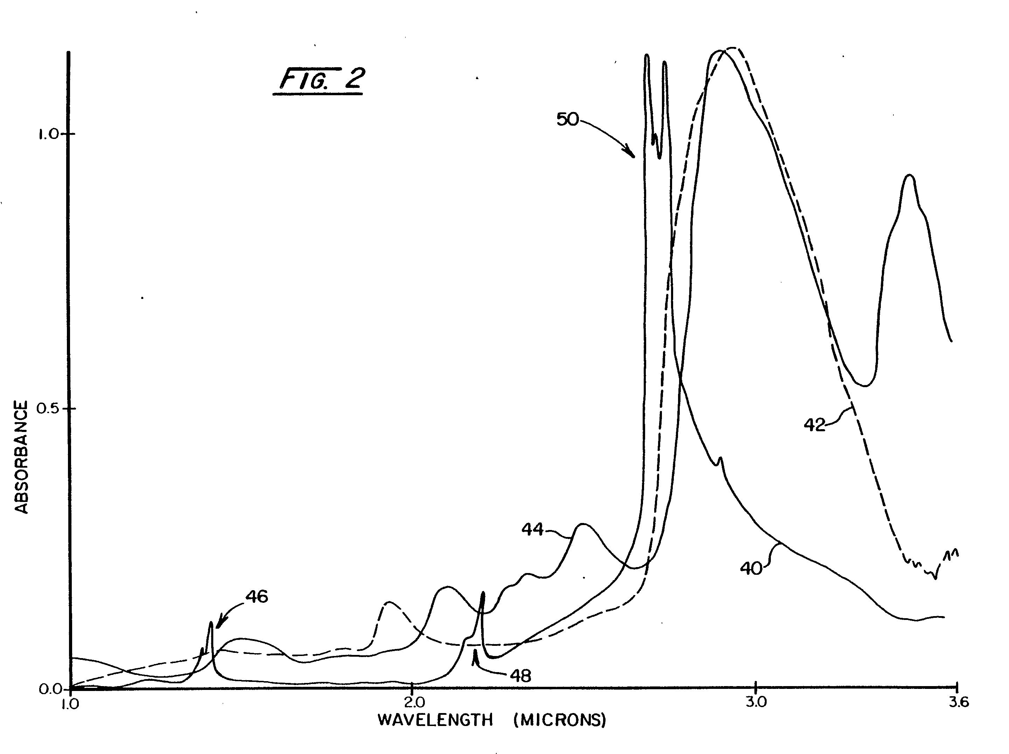

Referring to Fig. 2 which illustrates absorption curves 40,42,44 for kaolin, water, and cellulose, it can be seen that kaolin has peak absorption ranges 46,48,50 centered at about 1.4, 2.2, and 2.7 microns. The center wavelength of the kaolin measurement band is preferably selected from the 1.4-micron or 2.2-micron ranges 44,48 because the 2.7-micron range 50, although more sensitive to absorption by kaolin, does not offer much opportunity to balance the effects of absorption by water. The high absorptance of fiber at wavelengths above approximately 2.77 microns may necessitate selection of the kaolin reference band from among shorter wavelengths (e.g., a band centered at about 2.5 microns). This in turn would necessitate correction for absorption by water. Moreover, the 2.7-micron range 50 is so sensitive to absorption by kaolin that its use would be restricted to those sensors which are applied to the measurement of lighter grades of paper (e.g., paper having a weight of less than 40 grams per square meter).

In contrast, the 1.4-micron range 46 provides the opportunity to balance the effects of absorption by water by selecting, for example, a center wavelength of about 1.40 microns for the kaolin measurement band and a center wavelength of about 1.50 microns for the kaolin reference band. The 2.2-micron range 48 has the same advantage to a greater extent. Moreover, the 1.4-micron range 46 is less sensitive to absorption by kaolin than the 2.2-micron range 48, and has the further disadvantage of being in peak absorption ranges for water and fiber. Thus, the 2.2-micron range 48 will provide the best results in most cases.

A second filter 32 passes a first narrow band of reference radiation which in comparison to the kaolin measurement band is insensitive to absorption by kaolin. The center wavelength of the kaolin reference band is preferably selected so that sensitivity to absorption by water is substantially the same for both the kaolin measurement and kaolin reference bands. Accordingly, the second filter 32 preferably passes a band of radiation centered at about 1.87 microns.

Although the kaolin measurement and reference bands are selected to minimize differences in sensitivity to absorption by water, they may have significant differences in sensitivity to absorption by the fiber (cellulose) component of the sheet 8. Indications of kaolin content may be derived solely from the transmittances of the kaolin measurement and reference bands. However, the precison of these indications will vary with the uniformity of fiber content. To compensate for this effect, the third and fourth filters (not shown) are provided to derive indications of fiber content. This enables measurement of the dry kaolin content of the sheet 8 in a manner which corrects for nonuniformity of fiber content. In addition, by selecting the fiber measurement and fiber reference bands so that they have substantially the equal sensitivity to absorption by water, errors in the indications of kaolin content resulting from differential absorption by water become negligible.

Accordingly, the third and fourth filters are selected to pass fiber measurement and fiber reference bands centered at about 2.12 and 1.89 microns. respectively.

In operation, the selected narrow bands of radiation are emitted from the source 22, time-multiplexed via the rotating filter wheel 26, and directed to the sheet 8. Consequently, the detector 24 produces a separate voltage response (indicated by line 52) for each selected narrow band of radiation. Each such response 52 is indicative of the intensity of radiation transmitted through the sheet 8 for the narrow band of radiation with which the response is associated.

The transmittance of any particular band of radiation is typically represented mathematically as "I/I(0)", where "I" is a representation of the detector response 52 obtained during normal operation and "I(0)" is a stored representation of the detector response obtained during a standardization procedure in which the sheet 8 is not present in the pass gap 54.

The detector responses 52 are communicated to a conventional signal processing system (indicated by the dashed line 58) which typically includes a conventional ratio analyzer 60 and a computer 62. The signal processing system 58 employs the detector responses 52 to calcuate measurements of the dry weight percentage (or fraction) of the sheet 8 accounted for by kaolin. The calculations may be represented mathematically by the following equations. First,

$$X/Y - 1 = a(K + jC)(f(s)), \qquad (1)$$

where X is the transmittance of the kaolin reference band; Y is the transmittance of the kaolin measurement band; "a" is a calibration constant; K is the weight per unit area of kaolin contained in the sheet 8; C is the weight per unit area of fiber contained in the sheet ; "j" is a multiplier accounting for partial sensitivity to fiber; and "f(s)" is an error function that depends on physical properties of the kaolin and fiber (such as diameter and length) which will affect their optical scattering capabilities. Second,

$$V/Z - 1 = b(C + hK)(f(s), \qquad (2)$$

where V is the transmittance of the fiber reference band; Z is the transmittance of the fiber measurement band; "b" is a calibration constant; "f(s)" is the error function as in equation 1; and "h" is a multiplier accounting for partial sensitivity to kaolin. Third,

$$T = a(K + jC) / b(C + hP); \qquad (3)$$

where T is the ratio (X/Y - 1) / (V/Z 1) and is therefore substantially independent of the error function "f(s)" but is dependent on "hK". The combined weight of the kaolin and fiber is equal to the sum K + C. Therefore, equation 3 may be rewritten as follows:

$$T = (a/b)[\%K + j(\%C)] / [\%C + h(\%K)]; \qquad (4)$$

where %K is kaolin fraction or percentage, and %C is the fiber fraction or percentage, of the dry weight of the sheet 8. Since %C = 1 - %K,

$$T = (a/b)(\%K + j - j(\%K)) / (h(\%K) + 1 - \%K). \qquad (5)$$

Letting "a/b" equal d,

$$\%K = (dj - T) / [T(h - 1) - d(1 - j)]. \qquad (6)$$

In calibrating the sensor, an iterative computer program is used to determine the values for the calibration data d, j, and h which make equation 6 best fit laboratory-determined kaolin fraction values for a number of actual samples of the paper product being measured.

The kaolin fraction values are compared to a target value stored in the computer 62. In response to a deviation from the target value, control signals 64 are communicated from the computer 62 to a process control actuator 66, which may be any conventional actuator for controlling the amount of kaolin delivered to the headbox 68.

While the invention has been described according to the preferred embodiment, it is clear that numerous modifications can be made without departing from the spirit and of the invention. For example, although some means for modulating radiation emitted from the source will be desirable in any design, the combination of a filter wheel 26 with a single detector 24 can be replaced with plural detectors or multiple-channel detectors in which optical filters corresponding to the selected narrow bands are installed. In addition, although a transmission geometry is indicated and preferred in order to avoid error which may result from layering of the clay, the sensor can be modified to employ a reflection geometry. Thus, the above description is not intended to restrict the scope of the invention beyond that defined by the following claims and their equivalents.

## Claims

1. A sensor for use in measuring the amount of clay contained in a moving sheet of paper product, comprising:
a source housing containing a radiation source that emits infrared radiation over a spectral band which includes a plurality of selected narrow bands, the plurality including a first band of measurement radiation having a first center wavelength selected from a peak absorption range for kaolin and a first band of reference radiation having a second center wavelength selected from outside a peak absorption range for kaolin;
a detector housing for receiving radiation emitted from the source and transmitted through the sheet; and
means responsive to each selected band of radiation for producing separate electrical indications for each selected band of the intensity of radiation transmitted through the sheet.

2. A sensor as in claim 1 wherein the peak absorption range from which the first center wavelength is selected extends from 2.15 to 2.22 microns.

3. A sensor as in claim 1 wherein the peak absorption range from which the first center wavelength is selected extends from 1.37 to 1.42 microns.

4. A sensor as in claim 2 wherein the first center wavelength is absorbed by water to substantially the same degree as the second center wavelength.

5. A sensor as in claim 3 wherein the first center wavelength is absorbed by water to substantially the same degree as the second center wavelength.

6. A sensor as in claim 4 wherein the plurality also includes a second band of measurement radiation which is sensitive to absorption by cellulose fiber and a second band of reference radiation which is less sensitive to absorption by cellulose fiber.

7. A sensor as in claim 5 wherein the plurality also includes a second band of measurement radiation having a center wavelength which is sensitive to absorption by cellulose fiber and a second band of reference radiation having a center wavelength which is less sensitive to absorption by cellulose fiber.

8. A sensor as in claim 6 wherein the center wavelength of the second band of reference radiation is absorbed by water to substantially the same degree as the center wavelength of the second band of measurement radiation.

9. A sensor as in claim 7 wherein the center wavelength of the second band of reference radiation is absorbed by water to substantially the same degree as the center wavelength of the second band of measurement radiation.

FIG. 1

FIG. 2